# EUROPEAN PATENT SPECIFICATION

(11) **EP 3 227 666 B1**
(45) Date of publication and mention of the grant of the patent: **06.02.2019**
(21) Application number: 15816315.4
(22) Date of filing: 03.12.2015
(51) Int. Cl.: G01N 21/77, G01N 33/00

(54) **FILM BASED CARBON DIOXIDE SENSOR**
FILMBASIERTER KOHLENDIOXIDSENSOR
CAPTEUR DE DIOXYDE DE CARBONE À BASE DE FILM

(30) Priority: 04.12.2014 US 201462087345 P
(43) Date of publication of application: 11.10.2017
(73) Proprietor: Carrier Corporation, Farmington, CT 06489 (US)
(72) Inventor: DESMARAIS, Richard, Beverly, Massachusetts 01915 (US); VARGA, Stephen, Beverly, Massachusetts 01915 (US); LESHUK, Jeffrey Allen, Beverly, Massachusetts 01915 (US)
(74) Representative: Dehns
(86) International application number: PCT/US2015/063695
(87) International publication number: WO 2016/090117

(56) References cited:
- FR-A- 1 123 364
- US-A- 3 114 610
- US-A1- 2012 003 916
- US-A1- 2012 039 767
- US-A1- 2012 276 647
- US-A1- 2013 236 980

## Description

### BACKGROUND OF THE INVENTION

The embodiments herein generally relate to gas content sensors and more particularly to film based carbon dioxide sensors.

The detection of concentrations of gases in ambient air can provide various information regarding products or other items in an environment. Further, it may be desirable to maintain specific concentrations of gases in the ambient air of an environment in order to achieve beneficial, or non-detrimental, environmental impacts on products within the environment.

For example, increased carbon dioxide atmospheres are often used to help maintain the freshness of fruits, vegetables, ornamentals, and meat. If the CO₂ concentration is too low, the desired benefit may not be attained. Likewise, if the CO₂ concentration is too high, the product may be damaged. Thus, monitoring the levels of CO₂ in an environment is desirable. The concentration of CO₂ may be increased or decreased intentionally, and, in some cases, no increase in CO₂ over that of normal atmosphere may be desired or intended, i.e., maintaining the CO₂ levels at normal atmospheric levels. However, in the case of products that produce CO₂ from respiration, the CO₂ level may unintentionally increase, i.e., the concentration may accumulate to levels that may damage the products, such as fruits, vegetables, ornamentals, and meat, merely because of the natural CO₂ generation by the products.

Thus, detecting the concentrations of carbon dioxide may be beneficial to monitoring the status and quality of produce, fruits, vegetables, ornamentals, meats, and other perishable products ("products) in transport containers or in storage. As noted, the emission of CO₂ from these products can impact the state of the product. For example, during ripening conditions of products, such as bananas, emission of CO₂ from the product may increase the CO₂ concentration in the environment to undesirable levels. Similarly, microbial action in products during fermentation or spoilage may also generate CO₂ and may raise ambient concentrations in the environment to undesirable levels. Thus, measuring the CO₂ concentration present in an environment is desirable and allows the processes to be monitored non-invasively and in real-time, such that undesirable levels may be avoided.

Gas concentration sensors can be used to effectively measure the CO₂ content in the environment around the products in cold chain applications. Cold chain applications refer to refrigeration of foods, perishable items, and other products during transportation and storage. For example, cold chain applications may include storage and/or transportation of products in refrigerated storage facilities, refrigerated transport vehicles (especially refrigerated marine containers), sealed pallets, sealed boxes, and sealed packages.

Current sensing devices on the market use sophisticated and expensive CO₂ sensors that are typically based on non-dispersive infrared technology. This technology is electro-mechanically sophisticated and expensive. The devices have long device life and provide very accurate readings and detections of the concentrations of gases. However, the precision and device life duration of these sensing devices is not required in cold chain applications, particularly in view of the high cost of the sensing devices.

US2013/0236980 discloses a CO2 gas sensor based on a wavelength discrimination sensor.

### BRIEF DESCRIPTION OF THE INVENTION

According to a first aspect, the present invention provides a gas concentration monitoring system comprising: a radiation source comprising one or more emitting elements configured to emit radiation; a radiation sensor comprising a plurality of sensing elements configured to detect radiation received at the radiation sensor from the radiation source; and a reactive material located between the radiation source and the radiation sensor and configured to react to the presence of a gas, wherein the reaction of the reactive material impacts an amount of radiation at different wavelengths that passes through the reactive material, characterised in that the plurality of sending elements are arranged in parallel as an array in order to detect the different wavelengths passed through the reactive material; and an analog to digital converter operably connected to the array of radiation sensing elements, the analog to digital converter configured to sum the analog signals from the sensing elements and then convert the sum to a digital signal.

The monitoring system may include an enclosure having one or more vents configured to permit ambient air to enter the enclosure, the enclosure configured to house the radiation source, the radiation sensor, and the reactive material.

The reactive material may be a carbon dioxide color-sensitive film.

The radiation source may comprise one or more LEDs.

The impact on the amount of radiation detected at the radiation sensor may be one of (i) decreasing the amount of radiation that is received at the radiation sensor and (ii) increasing the amount of radiation that is received at the radiation sensor.

The monitoring system may include a control circuit configured to at least one of (i) control the radiation source and (ii) receive information from the radiation sensor.

The monitoring system may include a data logger configured to at least one of (i) record data associated with the amount of radiation received at the radiation sensor and (ii) determine if an amount of radiation received at the radiation sensor is either above or below a threshold.

The gas may be carbon dioxide.

According to a second aspect the present invention provides a method of monitoring a concentration of gas within an enclosure, the method comprising: controlling a radiation source to transmit radiation; passing the transmitted radiation through a reactive material; detecting the amount of radiation that passes through the reactive material with a plurality of sending elements arranged in parallel as an arrange in order to detect different wavelengths of radiation passing through the reactive material; using an analog to digital converter, summing and converting the signal from the array of sensing elements, determining a concentration of a gas based on the amount of radiation detected, at the different wavelengths, wherein the reactive material is configured to react to the presence of the gas, wherein the reaction of the reactive material impacts the amount of radiation at the different wavelengths that passes through the reactive material.

The method may include passing ambient air over the reactive material.

The gas may be carbon dioxide.

The radiation may be at least one of visible light, ultraviolet radiation, and infrared radiation.

The method may include logging the determined concentration of gas with a data logger.

Technical effects of at least the preferred embodiments of the invention include providing a low cost, simple system and sensor to detect the presence and content levels of carbon dioxide in an environment.

### BRIEF DESCRIPTION OF THE DRAWINGS

The subject matter which is regarded as the invention is particularly pointed out and distinctly claimed in the claims at the conclusion of the specification. The foregoing and other features and advantages of the invention are apparent from the following detailed description taken in conjunction with the accompanying drawings in which:
FIG. 1 is a schematic illustration of a carbon dioxide sensor system in accordance with an exemplary embodiment of the invention; and
FIG. 2 is a flow chart of a process in accordance with an exemplary embodiment of the invention.

### DETAILED DESCRIPTION OF THE INVENTION

As noted above, the monitoring and detection of carbon dioxide concentrations in cold chain applications assists in the monitoring of the quality of a product within cold chain applications. Cold chain applications include the transportation and storage of refrigerated, perishable products, such as produce and meats. With respect to fruits and vegetables, appropriate environmental concentrations of CO₂, e.g., within a shipping container, may range between 1% and 30%, depending on the specific product. Thus, it is important to monitor the levels of CO₂ to ensure that these appropriate levels are not exceeded, which may indicate that the food is approaching expiration or has expired.

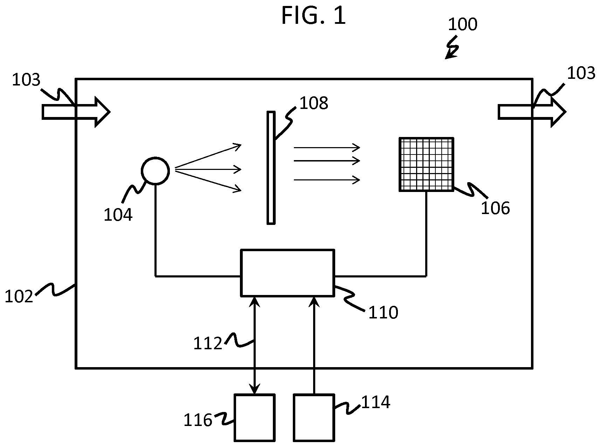

Referring to FIG. 1, a gas concentration monitoring system 100 in accordance with an exemplary embodiment of the invention is shown. The monitoring system 100 may be located within a container that is used for the transportation or storage of perishable products. For example, the container may be a refrigerated shipping container, a storage room, or a transportation vehicle. Further, in some embodiments, the monitoring system 100 may be located within the packaging of products or within a pallet that is used for transporting products.

The monitoring system 100 includes a sensor enclosure 102 that is configured to house various component parts of the monitoring system 100 and is exposed to ambient air (air external to the enclosure 102) through air vents 103. The air vents 103 allow for ambient air to flow and pass into and through the enclosure 102 such that the flowing ambient air interacts with sensing components of the monitoring system 100.

The sensing components of the monitoring system 100 include a radiation source 104, a radiation sensor 106, and a reactive material 108 located between the radiation source 104 and the radiation sensor 106. The radiation source 104 and the radiation sensor 106 are electrically connected to control circuity 110. The control circuitry 110 is configured to control and send commands to the radiation source 104 and receive and/or process information received from the radiation sensor 106.

The radiation source 104, for example, is configured as a pulse modulated single or multi-color LED that emits radiation in one or more spectra. The radiation source 104 is configured to emit radiation toward the radiation sensor 106. Those skilled in the art will appreciate that various electromagnetic radiation sources may be used without departing from the scope of the invention. For example, radiation sources may include sources of visible light radiation, ultraviolet radiation, infrared radiation, and/or combinations of these or other radiation spectra. As such, the radiation source 104 may be any type of radiation source known or will become known without departing from the scope of the invention.

Based on the type of radiation source 104 (and associated emitted radiation), the radiation sensor 106 is configured to detect radiation of the type generated by the radiation source 104. The radiation sensor 106 is an array of radiation sensors configured in parallel to detect the wavelength(s) and energy of the radiation emitted by the radiation source 104. In this embodiment, the radiation sensor 106 is configured to detect or measure the amount of radiation that is received at the elements of the radiation sensor 106 based on the energy received at the sensor elements. The sensor 106 will then output an analog or digital signal that is sent to the control circuitry 110 for further processing. The radiation sensor 106 consists of multiple sensing elements configured in parallel that are individually sensitive to specific, different wavelength. Thus, those skilled in the art will appreciate that the number of sensing elements is variable.

The reactive material 108 located between the radiation source 104 and the radiation sensor 106 is configured to affect the amount of radiation (and energy) that passes therethrough and reaches the sensor 106. For example, the reactive material 108 is configured to react to the presence of a gas in the ambient air, such as CO₂, and change or alter such that the amount of radiation that passes through the reactive material 108 is changed (either increased or decreased) relative to when no CO₂ or a baseline concentration of CO₂ is present. The change in received radiation at the radiation sensor 106 may be directly proportional to the change in the amount or concentration of CO₂ present in the enclosure 102. Thus, the concentration of a gas, such as CO₂, may be effectively and efficiently monitored and is indicative of the CO₂ concentration within the environment within which the monitoring system 100 is located.

The reactive material 108 may be a plastic, composite, glass, or other material that is inherently reactive to the presence of certain gas(es), such as CO₂, or may be formed of material that is combined with dyes, inks, pigments, or other compounds and/or materials to make a resultant material that is reactive to the presence of certain gas(es), such as CO₂. In some embodiments, the reactive material 108 may be configured to be transparent when there is no CO₂ present, when in the presence of atmospheric concentrations of CO₂, or when some predetermined concentration, threshold concentration, known concentration, or baseline concentration of CO₂ is present. Then, as the CO₂ concentration increases in the ambient air, the reactive material 108 will become opaque or change color, for example, and further transition to higher levels of opacity as the CO₂ concentration increases in the ambient air. Thus, the amount of radiation that passes through the reactive material 108 will lessen due to the absorption of the radiation by the opaque reactive material 108. As a result, the amount of radiation that reaches and is detected at the radiation sensor 106 will decrease and can be used to indicate changes in CO₂ concentrations.

As the color or opacity of the reactive material 108 changes, the reactive material 108 will absorb more of the radiation transmitted by the radiation source 104, thus decreasing the amount of radiation or energy received and detected by the radiation sensor 106. In other exemplary embodiments, the reactive material 108, as it reacts to the presence of CO₂, may instead reflect a greater portion of the radiation that is incident to the reactive material 108, or may disperse, or provide other impacts on the radiation that is transmitted from the radiation source 104, thus reducing the amount of energy received or detected by the radiation sensor 106. In these exemplary configurations, an increase in the presence of CO₂ will reduce the amount of energy received by the radiation sensor 106.

In some such embodiments, the reactive material 108 may be configured as a color changing film. In this embodiment, in the presence of CO₂, the color changing film (reactive material 108) will change color or change the opacity of a color. The color or opacity changing of the reactive material 108 may be a result of direct contact or interaction with CO₂ or may be due to an indirect interaction, such as pH levels of the moisture content in the ambient air that flows through the enclosure 102. The color of the color changing film is configured to match the radiation source 104 so that changes in the color changing film affect the amount of radiation that reaches radiation sensor 106.

In alternative embodiments, the reactive material 108 may operate in the opposite manner. For example, the reactive material 108 may be an opaque color when the concentration of CO₂ is low, and may become more transparent as the CO₂ concentration increases. In this configuration, the amount of energy or radiation detected by radiation sensor 106 will increase as the CO₂ concentration increases.

In accordance with embodiments of the invention, the change in detected radiation or energy by the radiation sensor 106 is indicative of the concentration of CO₂, or indicative of a change (a delta) in the concentration of CO₂, that is present within the sensor enclosure 102 and thus representative of the CO₂ content in the environment in which monitoring system 100 is located. As an example, the sensitivity of detecting the concentration of CO₂ may be to an accuracy of ±0.5% concentration within the range of 1% to 30% concentration of CO₂ in the ambient air. Thus, the monitoring system 100 can monitor the concentrations and/or changes in the concentrations of CO₂ to an adequate degree. Such accuracy is sufficient for the purpose of cold chain applications.

In the monitoring system 100, the detected radiation or energy at the sensor 106 is converted from an analog signal to a digital signal. The detected signals from the array of sending elements are summed and then converted to a digital signal. An analog-to-digital converter is provided within the system and may be part of the radiation sensor 106, part of the control circuitry 110, or part of some other connected and/or associated system or device. The digital signal may then be processed, recorded, and/or analyzed.

The control circuitry 110, as noted above, is in electrical communication with the radiation source 104 and the radiation sensor 106. The control circuitry 110 is configured to control the radiation source 104 to emit radiation upon command, which in some embodiments may allow for ON and OFF states of the radiation source 104. The ON/OFF states may be used to conserve power and/or enable data collection only when desired, without a continuous source of radiation. Further, as noted above, the control circuitry 110 may include the A/D converter to convert an analog signal received from the radiation sensor 106 to a digital signal for processing. As shown, the control circuitry 110 is located and/or housed within the enclosure 102. However, this is merely presented for exemplary purposes and those skilled in the art will appreciate that the control circuitry may be configured outside of the enclosure 102 and/or may be configured as part of a larger or more comprehensive computing device (e.g., a data logger 116 discussed below). Further, in some embodiments the digital signal may be a digital timing signal with frequency, voltage changes, pulse width output, or other outputs known or that will become known.

The control circuitry 110, as shown in FIG. 1, is further connected to a data interface 112 and a power source 114. The power source 114 may be a battery or other electrical power source known in the art, and may be configured to provide power to the monitoring system 100 and may also provide power to the data logger 116 and/or other electrical equipment.

The data interface 112 is configured to enable communication between the control circuitry 110 and other components, such as a data logger 116. The data logger 116 may include a central processing unit and volatile and non-volatile memory for data processing, data logging, control of alert and notification systems, input/output functions, etc. In some embodiments, the data logger 116 is configured as a data logging system which incorporates the monitoring system 100 along with other types of sensors and monitoring devices, such as humidity sensors, temperature sensors, etc.

The data logger 116 or other processing component can process and log the information collected at the radiation sensor 106 in order to monitor the CO₂ levels in the ambient air surrounding the monitoring system 100. The data logger 116 may be configured to monitor for specific percentage levels of CO₂ concentrations and/or monitor for a threshold level being surpassed in the ambient air. For example, if a threshold level is exceeded, it may indicate that the monitored products that are generating the CO₂ have reached a level that begins to indicate, for example, spoilage. In this case, the food may not have spoiled, but a particular threshold may indicate a level at which spoilage may become a concern. As such, the data logger 116 may monitor a time elapsed since the threshold has been exceeded. The monitored time may then be used to indicate if the product has reached or exceeded unacceptable levels or standards for the particular product.

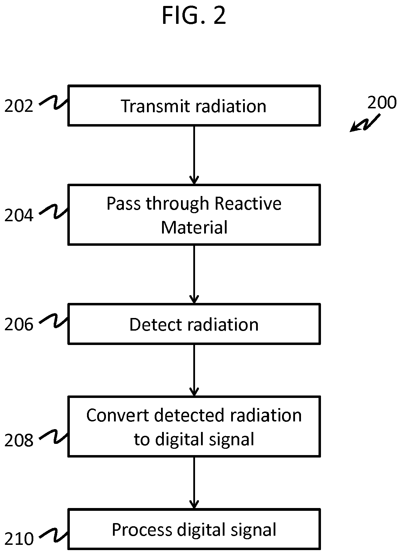

Turning now to FIG. 2, a flow chart of an exemplary process 200 is shown. As a first step 202, radiation is provided in an environment from a radiation source, such as an LED. The radiation is configured to pass through a reactive material at step 204 and be received or detected at a radiation sensor in step 206. The reactive material employed at step 204 is a color changing material such as described above with respect to reactive material 108 and the sensor is configured to detect the type of radiation transmitted by the radiation source. The sensor will detect or receive an amount of energy based on the amount of radiation that passes through the reactive material. An analog signal representative of the amount of energy detected at the sensor will then be converted to a digital signal at step 208 which will then be processed at step 210. The processing of the digital signal at step 210 may include logging the digital signal as a form of data and/or determining if the level of energy detected exceeds or is below a predetermined threshold. If the threshold is passed, either above or below depending on the configuration, a clock may start and/or an alarm or alert may be issued to indicate that the threshold has been passed.

Advantageously, embodiments of the invention provide a low cost CO₂ sensor that can affordably and effective be used in cold chain applications. A CO₂ sensor device or system, as disclosed herein, functions at adequate resolutions for cold chain applications, and thus provides a low cost alternative to overly accurate and expensive devices. Further, the sensor devices disclosed herein provide a single-use sensor that may be usable to monitor CO₂ levels over the course of weeks, and then be discarded. However, such configuration is advantageous because the cost is significantly low, particularly compared to high cost, precise, and long duration (order of years) sensors.

While the invention has been described in detail in connection with only a limited number of embodiments, it should be readily understood that the invention is not limited to such disclosed embodiments. Rather, the invention can be modified to incorporate any number of variations, alterations, substitutions, combination, sub-combination, or equivalent arrangements not heretofore described, but which are commensurate with the scope of the invention as defined by the claims. Additionally, while various embodiments of the invention have been described, it is to be understood that aspects of the invention may include only some of the described embodiments.

For example, although described herein as a reactive material located between a radiation source and a radiation sensor and thus transparent, in alternative embodiments, the same concepts may be applied to reflective materials, wherein the reflective properties of the reactive material changes based on the concentration of CO₂. In such embodiments, the sensor may be appropriately located and/or configured within the enclosure of the system. Moreover, in some embodiments, the reactive material may be directly coated onto the surface of the radiation sensor, such that, with reference to FIG. 1, elements 106 and 108 comprise a single element. Alternatively, the reactive material may be coated onto the surface or exterior of the radiation source, such that elements 104 and 108 are combined to form a single element. Thus, the configuration of FIG. 1 is merely presented for exemplary and explanatory purposes and does not limit the scope of the invention.

Further, although described with respect to the detection and monitoring of carbon dioxide, those skilled in the art will appreciate that the reactive material described herein may be configured to detect and/or react to the presence of other gases, compounds, and/or fluids, and thus the invention is not merely limited to detecting and monitoring carbon dioxide. Furthermore, in line with this, although described with respect to application within cold chain applications, those skilled in the art will appreciate that the claimed monitoring systems and methods may be used in other applications for the detection and monitoring of gases, or other fluids, in an environment.

Further, for example, the control circuitry and/or data logger disclosed above has been described as physically connected with the sensor components. However, those of skill in the art will appreciate that wireless communication may be used to provide the communication between the various components of the devices. Moreover, although described as separate elements, the sensor, the control circuitry, and the data logger may all, or some sub-combination thereof, be formed as a single unit.

Accordingly, the invention is not to be seen as limited by the foregoing description, but is only limited by the scope of the appended claims.

## Claims

1. A gas concentration monitoring system comprising:
a radiation source (104) comprising one or more emitting elements configured to emit radiation;
a radiation sensor (106) comprising a plurality of sensing elements configured to detect radiation received at the radiation sensor from the radiation source; and
a reactive material (108) located between the radiation source and the radiation sensor and configured to react to the presence of a gas, wherein the reaction of the reactive material impacts an amount of radiation at different wavelengths that passes through the reactive material,
wherein the plurality of sensing elements are arranged in parallel as an array in order to detect the different wavelengths passed through the reactive material, **characterized in that**
an analog to digital converter is operably connected to the array of radiation sensing elements, the analog to digital converter configured to sum the analog signals from the sensing elements and then convert the sum to a digital signal.

2. The monitoring system of claim 1, further comprising an enclosure (102) having one or more vents (103) configured to permit ambient air to enter the enclosure, the enclosure configured to house the radiation source (104), the radiation sensor (106), and the reactive material (108).

3. The monitoring system of any of the preceding claims, wherein the reactive material (108) is a carbon dioxide color-sensitive film.

4. The monitoring system of any of the preceding claims, wherein the radiation source (104) comprises one or more LEDs.

5. The monitoring system of any of the preceding claims, wherein the impact on the amount of the different wavelengths of radiation detected at the radiation sensor (106) is one of (i) decreasing the amount of radiation that is received at the radiation sensor and (ii) increasing the amount of radiation that is received at the radiation sensor.

6. The monitoring system of any of the preceding claims, further comprising a control circuit (110) configured to at least one of (i) control the radiation source (104) and (ii) receive information from the radiation sensor (106).

7. The monitoring system of any of the preceding claims, further comprising a data logger (116) configured to at least one of (i) record data associated with the amount of radiation received at the radiation sensor (106) and (ii) determine if an amount of radiation received at the radiation sensor is either above or below a threshold.

8. The monitoring system of any of the preceding claims, wherein the gas is carbon dioxide.

9. A method of monitoring a concentration of gas within an enclosure (102), the method comprising:
controlling a radiation source (104) to transmit radiation;
passing the transmitted radiation through a reactive material (108);
detecting the amount of radiation that passes through the reactive material with a plurality of sensing elements arranged in parallel as an array (106) in order to detect different wavelengths of radiation passing through the reactive material;
using an analog to digital converter, summing and converting the signals from the array of sensing elements, and
determining a concentration of a gas based on the amount of radiation detected, at the different wavelengths,
wherein the reactive material is configured to react to the presence of the gas, wherein the reaction of the reactive material impacts the amount of radiation at the different wavelengths that passes through the reactive material.

10. The method of claim 9, further comprising passing ambient air over the reactive material (108).

11. The method of claims 9 or 10, wherein the gas is carbon dioxide.

12. The method of any of claims 9, 10 or 11, wherein the radiation is at least one of visible light, ultraviolet radiation, and infrared radiation.

13. The method of any of claims 9-12, further comprising logging the determined concentration of gas with a data logger (116).

## Patentansprüche

1. Gaskonzentrationsüberwachungssystem, Folgendes umfassend:
eine Strahlungsquelle (104), die ein oder mehrere emittierende Elemente umfasst, die dazu konfiguriert sind, Strahlung zu emittieren;
einen Strahlungssensor (106), der eine Vielzahl von Sensorelementen umfasst, die dazu konfiguriert sind, Strahlung zu erkennen, die von der Strahlungsquelle an dem Strahlungssensor empfangen wird.
ein reaktives Material (108), das sich zwischen der Strahlungsquelle und dem Strahlungssensor befindet und dazu konfiguriert ist, auf das Vorhandensein eines Gases zu reagieren, wobei die Reaktion des reaktiven Materials Einfluss auf eine Menge von Strahlung in unterschiedlichen Wellenlängen nimmt, die das reaktive Material durchdringt,
wobei die Vielzahl von Sensorelementen parallel als eine Matrix angeordnet sind, um die unterschiedlichen Wellenlängen zu erkennen, die das reaktive Material durchdringen, **dadurch gekennzeichnet, dass**
ein Analog-Digital-Wandler betriebsmäßig mit der Matrix der Strahlungssensorelemente verbunden ist, wobei der Analog-Digital-Wandler dazu konfiguriert ist, die analogen Signale von den Sensorelementen zu summieren und dann die Summe in ein digitales Signal zu konvertieren.

2. Überwachungssystem nach Anspruch 1, ferner eine Umhüllung (102) mit einer oder mehreren Öffnungen (103) umfassend, die dazu konfiguriert sind, Umgebungsluft zu erlauben in die Umhüllung einzudringen, wobei die Umhüllung dazu konfiguriert ist, die Strahlungsquelle (104), den Strahlungssensor (106) und das reaktive Material (108) aufzunehmen.

3. Überwachungssystem nach einem der vorstehenden Ansprüche, wobei das reaktive Material (108) ein farbempfindlicher Kohlendioxidfilm ist.

4. Überwachungssystem nach einem der vorstehenden Ansprüche, wobei die Strahlungsquelle (104) eine oder mehrere LEDs umfasst.

5. Überwachungssystem nach einem der vorstehenden Ansprüche, wobei der Einfluss auf die Menge an unterschiedlichen Wellenlängen der Strahlung, die an dem Strahlungssensor (106) erkannt werden, eines von (i) Verringern der Menge an Strahlung, die an dem Strahlungssensor empfangen wird, und (ii) Erhöhen der Menge an Strahlung, die an dem Strahlungssensor empfangen wird, ist.

6. Überwachungssystem nach einem der vorstehenden Ansprüche, ferner einen Steuerkreislauf (110) umfassend, der zu mindestens einem von (i) Steuern der Strahlungsquelle (104) und (ii) Empfangen von Informationen von dem Strahlungssensor (106) konfiguriert ist.

7. Überwachungssystem nach einem der vorstehenden Ansprüche, ferner einen Datenlogger (116) umfassend, der zu mindestens einem von (i) Speichern von Daten, die der Menge an Strahlung, die an dem Strahlungssensor (106) empfangen wird, zugeordnet sind, und (ii) Bestimmen, ob eine Menge an Strahlung, die an dem Strahlungssensor empfangen wird, entweder oberhalb oder unterhalb eines Schwellenwertes liegt, konfiguriert ist.

8. Überwachungssystem nach einem der vorstehenden Ansprüche, wobei das Gas Kohlendioxid ist.

9. Verfahren zum Überwachen einer Gaskonzentration innerhalb einer Umhüllung (102), das Verfahren Folgendes umfassend:
Steuern eine Strahlungsquelle (104), um Strahlung zu übertragen;
Durchdringen eines reaktiven Materials (108) mit der übertragenen Strahlung;
Erkennen einer Menge an Strahlung, die das reaktive Material durchdringt, mit einer Vielzahl von Sensorelementen, die parallel als eine Matrix (106) angeordnet sind, um unterschiedliche Wellenlängen der Strahlung zu erkennen, die das reaktive Material durchdringt;
Verwenden eines Analog-Digital-Wandlers zum Summieren und Konvertieren der Signale von der Matrix der Sensorelemente, und
Bestimmen einer Gaskonzentration, basierend auf der Menge an erkannter Strahlung in den verschiedenen Wellenlängen,
wobei das reaktive Material dazu konfiguriert ist, auf das Vorhandensein von Gas zu reagieren, wobei die Reaktion des reaktiven Materials Einfluss auf die Menge an Strahlung in unterschiedlichen Wellenlängen nimmt, die das reaktive Material durchdringen.

10. Verfahren nach Anspruch 9, ferner das Vorbeiführen von Umgebungsluft über das reaktive Material (108) umfassend.

11. Verfahren nach Anspruch 9 oder 10, wobei das Gas Kohlendioxid ist.

12. Verfahren nach einem der Ansprüche 9, 10 oder 11, wobei die Strahlung mindestens eines von sichtbarem Licht, ultravioletter Strahlung und Infrarotstrahlung ist.

13. Verfahren nach einem der Ansprüche 9-12, ferner das Protokollieren der bestimmten Gaskonzentration mit einem Datenlogger (116) umfassend.

## Revendications

1. Système de surveillance de concentration de gaz comprenant :
une source de rayonnement (104) comprenant un ou plusieurs éléments émetteurs configurés pour émettre un rayonnement ;
un détecteur de rayonnement (106) comprenant une pluralité d'éléments de détection configurés pour détecter un rayonnement reçu au détecteur de rayonnement en provenance de la source de rayonnement ; et
un matériau réactif (108) placé entre la source de rayonnement et le détecteur de rayonnement et configuré pour réagir à la présence d'un gaz, dans lequel la réaction du matériau réactif impacte une quantité de rayonnement à différentes longueurs d'ondes qui passe à travers le matériau réactif,
dans lequel
la pluralité d'éléments de détection sont agencés en parallèle en tant qu'une matrice afin de détecter les différentes longueurs d'ondes qui sont passées à travers le matériau réactif, **caractérisé en ce que**
un convertisseur analogique-numérique est relié pour un fonctionnement à la matrice d'éléments de détection de rayonnement, le convertisseur analogique-numérique étant configuré pour totaliser les signaux analogiques en provenance des éléments de détection et convertir ensuite la somme en un signal numérique.

2. Système de surveillance selon la revendication 1, comprenant en outre une enceinte (102) pourvue d'un ou plusieurs évents (103) configurés pour permettre à l'air ambiant d'entrer dans l'enceinte, l'enceinte étant configurée pour abriter la source de rayonnement (104), le détecteur de rayonnement (106), et le matériau réactif (108).

3. Système de surveillance selon l'une quelconque des revendications précédentes, dans lequel le matériau réactif (108) est un film de dioxyde de carbone sensible à la couleur.

4. Système de surveillance selon l'une quelconque des revendications précédentes, dans lequel la source de rayonnement (104) comprend une ou plusieurs DEL.

5. Système de surveillance selon l'une quelconque des revendications précédentes, dans lequel l'impact sur la quantité des différentes longueurs d'ondes de rayonnement détectées au détecteur de rayonnement (106) est l'une de (i) une diminution de la quantité de rayonnement qui est reçu au détecteur de rayonnement et (ii) une augmentation de la quantité de rayonnement qui est reçue au détecteur de rayonnement.

6. Système de surveillance selon l'une quelconque des revendications précédentes, comprenant en outre un circuit de contrôle (110) configuré pour au moins l'un de (i) un contrôle de la source de rayonnement (104) et (ii) une réception d'informations en provenance du détecteur de rayonnement (106).

7. Système de surveillance selon l'une quelconque des revendications précédentes, comprenant en outre un enregistreur de données (116) configuré pour au moins l'un de (i) un enregistrement de données associées à la quantité de rayonnement reçu au détecteur de rayonnement (106) et (ii) une détermination du fait qu'une quantité de rayonnement reçu au détecteur de rayonnement est soit supérieure soit inférieure à un seuil.

8. Système de surveillance selon l'une quelconque des revendications précédentes, dans lequel le gaz est du dioxyde de carbone.

9. Procédé de surveillance d'une concentration de gaz à l'intérieur d'une enceinte (102), le procédé comprenant :
le contrôle d'une source de rayonnement (104) pour émettre un rayonnement ;
le passage du rayonnement émis à travers un matériau réactif (108) ;
la détection de la quantité de rayonnement qui passe à travers le matériau réactif avec une pluralité d'éléments de détection agencés en parallèle en tant qu'une matrice (106) afin de détecter différentes longueurs d'ondes de rayonnement qui passent à travers le matériau réactif ;
l'utilisation d'un convertisseur analogique-numérique, la totalisation et la conversion des signaux en provenance de la matrice d'éléments de détection, et
la détermination d'une concentration d'un gaz sur la base de la quantité de rayonnement détecté, aux différentes longueurs d'ondes,
dans lequel le matériau réactif est configuré pour réagir à la présence du gaz, dans lequel la réaction du matériau réactif impacte la quantité de rayonnement aux différentes longueurs d'ondes qui passe à travers le matériau réactif.

10. Procédé selon la revendication 9, comprenant en outre le passage d'air ambiant par-dessus le matériau réactif (108).

11. Procédé selon la revendication 9 ou 10, dans lequel le gaz est du dioxyde de carbone.

12. Procédé selon l'une quelconque des revendications 9, 10 ou 11, dans lequel le rayonnement est au moins l'un de la lumière visible, d'un rayonnement ultraviolet, et d'un rayonnement infrarouge.

13. Procédé selon l'une quelconque des revendications 9 à 12, comprenant en outre l'enregistrement de la concentration déterminée de gaz avec un enregistreur de données (116).
